# EUROPEAN PATENT APPLICATION

(11) **EP 0 948 956 A2**
(43) Date of publication of application: **13.10.1999**
(21) Application number: 99106331.4
(22) Date of filing: 26.03.1999
(51) Int. Cl.: A61K 6/083

(54) **Self-curing dental adhesive**

(30) Priority: 27.03.1998 US 79777 P
(71) Applicant: DENTSPLY DETREY GmbH, D-63303 Dreieich (DE)
(72) Inventor: Klee, Joachim E., Dr., 78315 Radolfzell (DE); Walz, Uwe, 78467 Konstanz (DE); Lu, Kawang, Dover, DE 19901 (US)
(74) Representative: Hartz, Nikolai F., Dr.

(57) **Abstract**

A self-curing dental adhesive has at least one polymerizable resin; at least one polymerizable monomer; at least one polymerizable monomer with an acidic moiety selected from the group 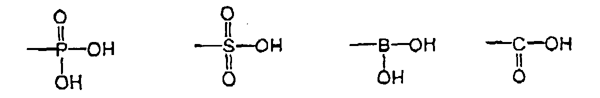 and salts thereof; a stabilizer; an organic solvent or water in a content of from about 10 to about 90 percent by weight; and, at least one part of a thermal redox initiator system or a photoinitiator. The adhesive polymerizes immediately after application of a self-curing dental material that comprises a redox-initiator system, and has an adhesion to dentine of at least about 7 MPa.

## Description

### Technical Field

The invention concerns a self-curing dental adhesive, which polymerizes after application of a self-curing dental restorative material that comprises a metal-based redox-initiator system without mixing of both systems.

### Background of the Invention

Presently, the dental treatment of cavities of the teeth usually includes using dental composites, compomers or glass ionomers. The use of dental composites and compomers is connected with the application of the adhesive technique. Commonly, both adhesive and dental restorative materials are cured by irradiation with visible light. Dental composites have numerous advantages: high mechanical strength, excellent esthetic properties and a high abrasion resistance. These positive properties are complemented by the fluoride releasing properties of compomers.

The use of glass ionomers is advantageous at least in part because they are self-adhesive, biocompatible and usable without requiring irradiation by light. Furthermore, they can release fluoride ions. Otherwise, glass ionomers have a relatively low flexural strength and a high abrasion.

In order to develop a self-curing restorative material with improved mechanical properties, high abrasion resistance and fluoride release a thermal setting adhesive is required.

Recently, a dual-curing adhesive comprising sulfinates and champhor quinone/amines as polymerization initiator was developed by Dentsply Caulk, Milford DE.

Generally, a self-curing material is composed of at least of two components, due to the fact that the parts of the initiator system must be distributed in these components.

### Summary of the Invention

It is an object of the present invention to provide a dental adhesive material.

It is another object of the invention to provide such a material that is self-curing.

These and other objects of the invention which shall become apparent from the present discussion and the following specification are accomplished by the invention as hereinafter described and claimed.

In general, a self-curing dental adhesive, comprises:
(i) at least a polymerizable resin
(ii) at least a polymerizable monomer
(iii) at least a polymerizable monomer with an acidic moiety selected from the group consisting of: 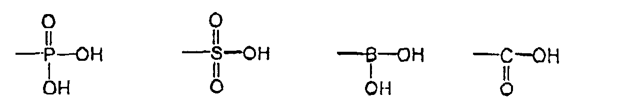 and salts thereof
(iv) at least a stabilizer,
(v) at least an organic solvent or water in a content of 10 to 90 percent weight of the adhesive and,
(vi) at least one part of a thermal redox initiator system or a photoinitiator,
wherein the dental adhesive is polymerizable immediately after application of a self-curing dental material that comprises a redox-initiator system, and wherein the dental adhesive has an adhesion to dentine of at least about 7 MPa.

### Preferred Embodiments for Carrying Out the Invention

The present invention includes a self-curing dental adhesive that comprises at least a polymerizable resin, a polymerizable monomer, a polymerizable monomer with at least an acidic moiety selected from the group 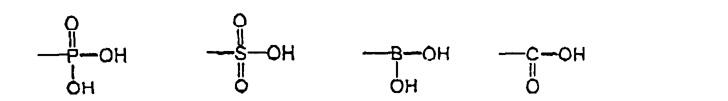 or salts thereof, a stabilizer, an organic solvent and/or water in a content of 10 to 90 percent, and at least one part of a thermal redox initiator system and/or a photoinitiator that polymerizes immediately after application of a self-curing dental material that comprises a redox-initiator system.

The adhesion of the invented self-curing adhesive is at least about 7 MPa, preferably 9 MPa, most preferably 11 MPa.

Preferably, the self-curing dental adhesive contains a salt of a metal or an organo-metalic compound, an amine or a peroxide. Usable metal salts or organo-metalic compounds are selected from the group consisting of copper, silver, cerium, iron, chromium, nickel, vanadium and manganese. Frequently, the metal containing compound is an acetate, salicylate, naphenoate, thiourea complex, acetylacetonate or ethylene tetramine acidic acid.

The preferred amines are alkyl aryl amines, dialkyl aryl amines, trialkyl amines or derivatives therefrom.

Preferred peroxides include for example, diacyl peroxides, peresters, perketales, peroxy dicarbonates, dialkyl peroxides, ketone peroxides or alkyl hydroxyperoxides, such as benzoylperoxide, 2,5-dimethyl-2,5-di(benzolyperoxy)hexane, tert.-butylamyl peroxide, di-(tert.-butyl) peroxide, cumenhydoperoxide, tert.-butylhydroperoxide, tert.-butylperoxy-(3,5,5-tri-methylhexanoate) and tert.-butylperoxy-2-ethylhexyl carbonate, laurylperoxide.

For example a dental adhesive comprising cupric acetylacetonate is applied in a cavity. The cavity is then filled by using a thermal setting material, that comprises cupric thiourea. Both materials polymerize together, whereby the self-curing restorative material also initiates the polymerization of the bonding material. The adhesion of this adhesive/restorative material is 9.0 ± 1.5 MPa.

### Comparative Example 1

### Dental adhesive

Equal amounts of Prime & Bond 2.1™ (Dentsply, De Trey, Constance) and the Self Cure Activator (Dentsply, Caulk, Milford DE) were mixed.

### Self-curing dental restorative material

The used dental restorative material is composed of a powder and a liquid. These have the following composition:

### Powder:

50.3639 g silylated Strontium-alumo-silicate glass, 2.1546 g silylated Strontium-alumo-silicate glass comprising 2 wt-% cupric thiourea complex and 1.0773 g silylated Strontium-alumo-silicate glass containing 10 wt-% ascorbic acid were mixed homogeneously.

### Liquid:

18.0000 g of dipentaerthrytrolpentaacrylate monophosphate and 2-(dimethyl) aminoethyl methacrylate (**AP-1**) comprising 30 wt-% diethylene glycol dimethacrylate, 4.5000 g of macromonomer **M-1** (synthesized by reaction of two mols of methacrylic acid, two mols of 2,2-bis-[4-(2,3-epoxypropoxy)-phenyl]-propane and one mole of adipic acid) comprising 30 wt-% diethylene glycol dimethacrylate), 10.6328 g of diethylene glycol dimethacrylate, 0.0167 g of N,N-bis(β-hydroxyethyl)-p-toluidine, 0.5049 g of tert.-butyl peroxy benzoate, 0.0113 g of 2,6-di-tert.-butyl-p-cresol were mixed homogeneously.

### Application

Immediately before use the dental adhesive is applied. Thereafter the restorative material obtained by homogeneous mixture of powder and liquid, in the weight (wt.)-ratio 3.2:1.0, was applied. The working time is 3.75 minutes and the setting time is 3.00 minutes. The adhesion to dentine was measured to be 8.58 ± 1.32 MPa. The dental restorative material shows the following mechanical properties: compressive strength of 256.6 ± 11.9 MPa, a flexural strength of 87.0 ± 3.4 and an E-modules of 7780 ± 785 MPa.

### Example 1

### Dental adhesive A

3.2419 g of 7,7,9,63,63,65-hexamethyl-4,13,60,69-tetraoxo-3,14,19,24,29,34,39,44,49,54, 59,70-dodecaneoxa-5,12,61,68-tetraaza doheptaconta diyl -1,72- dimethacrylate (R 5621), 3.7406 g of dipentaerythrol pentaacrylate monophosphate, 8.2819 g of 2,7,7,9,15-pentamethyl-4,13-dioxo-3,14-diox-5,12-diaza-hexadecanediyl-1,16-dimethacylate, 0.4417 g of bisphenol-A-dimethacrylat, 0.1496 g of cetylaminhydrofluorid, 0.0377 g of 2.6-di-tert.-butyl-p-cresol and 0.0330 g of cupric-II-acetylacetonate were dissolved in 59.9736 g of acetone.

### Self-curing dental restorative material

The dental restorative material is composed of powder and liquid as described in comparative example 1.

### Application

Immediately before use the dental adhesive is applied. Thereafter the restorative material obtained by homogeneous mixed of powder and liquid, in the wt.-ratio 3.2:1.0, was applied. The working time is 3.75 minutes and the setting time is 3.00 minutes. The adhesion to dentine was measured to be 9.0 ± 1.5 MPa. The dental restorative material shows the following mechanical properties: compressive strength of 256.6 ± 11.9 MPa, a flexural strength of 87.0 ± 3.4 and an E-modules of 7780 ± 785 MPa.

### Example 2

### Dental adhesive A

4.7593 g dipentaerythrol pentaacrylate monophosphate. 7.7083 g 2,7,7,9,15-pentamethyl-4,13-dioxo-3,14-diox-5,12-diazahexadecanediyl-1,16-dimethacylate, 2.2130 g of 7,7,9,63,63,65-hexamethyl-4,13,60,69-tetraoxo-3,14,19,24,29,34,39,44,49,54,59,70-dodecaneoxa-5,12,61 ,68-tetraaza doheptacontadiyl-1,72-dimethacrylate (R 5621), 1.3177 g trimethylolpropane triacrylate, 2.8213 g diethylene glycol dimethacrylate, 0.0997 g cuppric acetyl acetonate, 0.1922 g 2,6-di-tert.-butyl-p-cresol, 0.1922 g cetylaminhydrofluorid and 1.6952 g of a nanofiller (av. particle size 7 nm), were dissolved in 79.0011 g of acetone.

### Self-curing dental restorative material

The dental restorative material is composed of powder and liquid as described in comparative example 1.

### Application

Immediately before use the dental adhesive is applied. Thereafter the restorative material obtained by homogeneous mixed of powder and liquid, in the wt.-ratio 3.4:1.0, was applied. The working time is 3.25 minutes and the setting time is 3.00 minutes. The adhesion to dentine was measured to be 11.1 ± 1.8 MPa.

### Example 3

### Dental adhesive A

4.7137 g dipentaerythrol pentaacrylate monophosphate, 7.6345 g 2,7,7,9,15-pentamethyl-4,13-dioxo-3,14-diox-5,12-diazahexadecanediyl-1,16-dimethacylate, 2.1918 g of 7,7,9,63,63,65-hexamethyl-4,13,60,69-tetraoxo-3,14,19,24,29,34,39,44,49,54, 59,70-dodecaneoxa-5,12,61 ,68-tetraaza doheptacontadiyl-1,72-dimethacrylate (R 5621), 1.3051 g trimethylolpropane triacrylate, 2.7943 g diethylene glycol dimethacrylate, 1.0559 g p-dimethyl benzoic acid ethylester, 0.1904 g 2,6-di-tert.-butyl-p-cresol, 0.1904 g cetylaminhydrofluorid and 1.6790 g of a nanofiller (av. particle size 7 nm), were dissolved in 78.2450 g of acetone.

### Self-curing dental restorative material

The dental restorative material is composed of powder and liquid as described in comparative example 1.

### Application

Immediately before use the dental adhesive is applied. Thereafter the restorative material obtained by homogeneous mixed of powder and liquid, in the wt.-ratio 3.4:1.0, was applied. The working time is 3.25 minutes and the setting time is 3.00 minutes. The adhesion to dentine was measured to be 8.9 ± 2.6 MPa.

### Example 4

### Dental adhesive A

3.2419 g of 7,7,9,63,63,65-hexamethyl-4,13,60,69-tetraoxo-3,14,19,24,29,34,39,44,49,54, 59,70-dodecaneoxa-5,12,61,68-tetraaza doheptacontadiyl -1,72- dimethacrylate (R 5621), 3.7406 g of dipentaerythrol pentaacrylate monophosphate, 8.2819 g of 2,7,7,9,15-pentamethyl-4,13-dioxo-3,14-diox-5,12-diaza-hexadecanediyl-1,16-dimethacylate, 0.4417 g of bisphenol-A-dimethacrylat, 0.1496 g of cetylaminhydrofluorid, 0.0377 g of 2,6-di-tert.-butyl-p-cresol and 0.2283 g of cupric-II-acetylacetonate were dissolved in 59.9736 g of acetone.

### Self-curing dental restorative material

### Powder:

89.10 g silylated Strontium-alumo-silicate glass, 10-10 g OX-50 organic filler, 1.00 g Aerosil R-972, 0.02 g cupric acetylacetonate and 0.15 g ascorbyl palmitate were mixed homogeneously.

### Liquid:

10.00 g OEMA, 10.00 g dipentaerthrytrolpentaacrylate monophosphate, 30.00 g diethylene glycol dimethacrylate, 50.00 g 2,2-bis-[p-(2-hydroxy-3-methacryloyloxypropoxy)-pheny]-propane, 2.00 g distilled water and 0.20 g tert.-butyl peroxy benzoate were mixed homogeneously.

Apply one coat of the primer to etched dentine, air dry. Triturate powder and liquid of the restorative in capsule at 3.75:1 ratio for 12 seconds. Apply the paste to dentine in a post. Let the assembly self cure at 37°C. Then stored in 37°C water for 24 hours. Test sheer bond strength on Instron. The average bond strength was 9.18 Mpa.

It is evdident therefore, that the objects of a a self-curing dental adhesive are carried out by the invention as herein described. All possible aspects of the invention beyond the best mode have not been necessarily described, and the scope of the invention shall only be determined by the following claims.

## Claims

1. A self-curing dental adhesive, comprising
(i) at least one polymerizable resin;
(ii) at least one polymerizable monomer;
(iii) at least one polymerizable monomer with an acidic moiety selected from the group consisting of 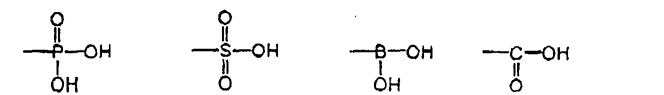 and salts thereof;
(iv) a stabilizer;
(v) an organic solvent or water in a content of from about 10 to about 90 percent; and,
(vi) at least one part of a thermal redox initiator system or a photoinitiator;
wherein the adhesive polymerizes immediately after application of a self-curing dental material that comprises a redox-initiator system, and has an adhesion to dentine of at least about 7 MPa.

2. A self-curing dental adhesive of claim 1 having an adhesion to dentine of at about 11 MPa.

3. A self-curing dental adhesive of claim 1 wherein said thermal redox initiator system is a salt of a metal or an organo-metalic cntaining compound, an amine or a peroxide.

4. A self-curing dental adhesive of claim 3 wherein said metal of said metal containing compound is selected from the group consisting of copper, silver, cerium, iron, chromium, nickel, vanadium and manganese.

5. A self-curing dental adhesive of claim 3 wherein said metal containing compound is an acetate, salicylate, naphenoate, thiourea complex, acetylacetonate or ethylene tetramine acidic acid.

6. A self-curing dental adhesive of claims 3 wherein said amine is is an alkyl aryl amine, a dialkyl aryl amine, a trialkyl amine or derivatives therefrom.

7. A self-curing dental adhesive of claims 3 wherein said peroxide is a diacyl peroxide, a perester, a perketale, a peroxy dicarbonate, a dialkyl peroxide, a ketone peroxide or a alkyl hydroxyperoxide.

8. A self-curing dental adhesive of claims 3 wherein said peroxide selected from benzoylperoxide, 2,5-dimethyl-2,5-di(benzolyperoxy)hexane, tert.-butylamyl peroxide, di-(tert.-butyl) peroxide, cumenhydoperoxide, tert.-butylhydroperoxide, tert.-butylperoxy-(3,5,5-tri-methylhexanoate) and tert.-butylperoxy-2-ethylhexyl carbonate, laurylperoxide.
